# EUROPEAN PATENT APPLICATION

(11) **EP 2 568 307 A1**
(43) Date of publication of application: **13.03.2013**
(21) Application number: 12182926.1
(22) Date of filing: 04.09.2012
(51) Int. Cl.: G01R 33/54, G01R 33/34

(54) **Improved patient positioning within an MRI device**

(30) Priority: 07.09.2011 KR 20110090710
(71) Applicant: Samsung Electronics Co., Ltd., Suwon-si, Gyeonggi-do, 443-742 (KR)
(72) Inventor: Lee, Ju Hyung, Gyeonggi-do (KR); Kwon, Oh Soo, Seoul (KR)
(74) Representative: Grünecker, Kinkeldey, Stockmair & Schwanhäusser

(57) **Abstract**

A magnetic resonance imaging (MRI) device and a control method thereof reduce the preparation time for MRI photographing a patient. A patient table has a transfer unit for moving a patient inside the magnet assembly. A fixing unit includes a signal transmitting unit for transmitting a signal such as an optical signal, an ultrasound signal or an RF signal. A signal receiving unit is installed on a diagnosis area of the patient or a radio frequency (RF) coil attached to a diagnosis area of the patient, and a patient position determination system calculates position information of the diagnosis area of the patient or the RF coil if the signal receiving unit detects a signal output from the signal transmitting unit when the transfer unit transfers the patient to inside the magnet assembly and to transfer the diagnosis area of the patient to an optimum photographic area inside the magnet assembly (i.e. the isocentre of the magnet assembly) based on the position information.

## Description

### BACKGROUND OF THE INVENTION

### 1. Field of the Invention

The present invention relates to magnetic resonance imaging, and in particular to a magnetic resonance imaging device used to diagnose various diseases by use of a magnetic resonance image and a control method thereof.

### 2. Description of the Related Art

In general, a medical imaging device provides an image obtained from a patient. The medical imaging device may include ultrasonic diagnostic equipment, x-ray tomography equipment, magnetic resonance imaging equipment, and medical diagnostic equipment. Typically, magnetic resonance imaging equipment functions under relatively less stringent conditions for photographing patients compared to other medical imaging devices while providing superior contrast images of soft tissues of a human body, as well as various types of diagnostic information; thereby having a high status in diagnostic technology using medical images.

Generally, a magnetic resonance imaging device includes imaging equipment that diagnoses internal structures of a human body using the energy - already converted to a signal - induced from resonance reactions obtained by applying a constant rate of frequency and energy to nuclei of atoms of a patient while a predetermined magnetic field is applied to the patient.

The magnetic resonance device requires preparatory time as well as photographing time for obtaining the images needed from a patient. Preparatory time represents a predetermined period of time consumed; for example, to move the patient from a hospital room to a magnetic resonance imaging (MRI) room; to move the patient to a patient table on the magnetic resonance imaging device in the MRI room; and to adjust the range of the images needed. Photographing time represents a predetermined period of time consumed to photograph a diagnosis area of the patient after completing the preparation.

A longer photographic time typically makes the patient feel tedious, and if the patient moves during the photographing time to adjust to the tedium, the quality of the obtained MRI images is degraded.

### SUMMARY OF THE INVENTION

Therefore, it is an aspect of the present invention to provide a magnetic resonance imaging device capable of reducing the preparation time for photographing a patient, and a control method thereof.

Additional aspects of the invention will be set forth in part in the description which follows and, in part, will be obvious from the description, or may be learned by the practice of the invention.

In accordance with one aspect of the present invention, a magnetic resonance imaging device which has a magnet assembly, a patient table provided with a transfer unit for introducing a patient to inside the magnet assembly and a fixing unit supporting the transfer unit, which includes a signal transmitting unit, a signal receiving unit, and a patient position determination system. The signal transmitting unit is installed on the magnet assembly. The signal receiving unit is installed on a diagnosis area of the patient or a radio frequency (RF) coil attached to a diagnosis area of the patient. The patient position determination system is configured to calculate position information of the diagnosis area of the patient or the RF coil if the signal receiving unit detects a signal output from the signal transmitting unit when the transfer unit transfers the patient to inside of the magnet assembly and to transfer the diagnosis area of the patient to an optimum photographic area inside the magnet assembly based on the position information of the diagnosis area of the patient or the RF coil.

The signal transmitting unit includes a plurality of signal transmitting units that are installed on the magnet assembly, and wherein if the signal receiving unit receives signals from the plurality of signal transmitting units, the patient position determination system calculates distances of the RF coil or the diagnosis area of the patient, which has the signal receiving unit installed thereon, with respect to each of the plurality of signal transmitting units, and calculates a position of the RF coil or the diagnosis area of the patient based on the calculated distance information.

If the position of the RF coil or the diagnosis area of the patient is calculated, the patient position determination system calculates a distance between the position of the RF coil or the diagnosis area of the patient and an optimum photographic area in the magnetic assembly and transfers the diagnosis area of the patient to the optimum photographic area in the magnet assembly.

The signal transmitting unit outputs a signal with a broad frequency band or a signal with a narrow frequency band, and if the signal transmitting unit is designed to output a signal with a narrow frequency band, the patient position determination system determines that the signal transmitting unit is disposed in line with the signal receiving unit along a direction of the magnet assembly at a point of time in which the signal receiving unit receives a signal.

If the signal transmitting unit is designed to output a signal with a broad frequency band, the patient position determination system determines that the signal transmitting unit is disposed in line with the signal receiving unit along the direction of the magnet assembly at a point of time in which intensity of a signal received by the signal receiving unit changes from increasing to decreasing.

In accordance with another aspect of the present invention, a control method of a magnetic resonance imaging device which has a magnet assembly and a patient table provided with a transfer unit introducing a patient to inside the magnet assembly and a fixing unit supporting the transfer unit is as follows. It is checked whether a signal transmitted from a signal transmitting unit installed on a predetermined area of the magnet assembly is received by a signal receiving unit, which is installed on a diagnosis area of the patient or a radio frequency (RF) coil attached to a diagnosis area of the patient, when the patient is transferred to the magnet assembly. If the signal transmitted from the signal transmitting unit is received by the signal receiving unit, a position of the diagnosis area of the patient or the RF coil is calculated based on information about the received signal. The diagnosis area of the patient is transferred to an optimum photographic area in the magnet assembly.

The signal transmitting unit includes a plurality of signal transmitting units installed on the magnet assembly. If the plurality of signal transmitting units is installed on the magnet assembly, distances of the signal receiving unit with respect to each of the plurality of signal transmitting units are calculated, and a position of the RF coil or the diagnosis area of the patient is calculated based on the calculated distance information.

If the signal transmitting unit outputs a signal with a narrow frequency band, it is determined that the signal transmitting unit is disposed in line with the diagnosis area of the patient or the RF coil along a length direction of the magnet assembly at a point of time in which the signal receiving unit receives a signal.

If the signal transmitting unit outputs a signal having a broad frequency band, it is determined that the signal transmitting unit is disposed in line with the diagnosis area of the patient or the RF coil along the length direction of the magnet assembly at a point of time in which intensity of a signal received by the signal receiving unit changes from increasing to decreasing.

In accordance with another aspect of the present invention, a magnetic resonance imaging device which has a magnet assembly and a patient table provided with a transfer unit introducing a patient to inside the magnet assembly and a fixing unit supporting the transfer unit includes a tag a tag terminal and a patient position determination system. The tag is installed on a predetermined area of the magnet assembly. The tag terminal is installed on a diagnosis area of the patient or a radio frequency (RF) coil attached to a diagnosis area of the patient. The patient position determination system is configured to calculate position information about the diagnosis area of the patient or the RF coil if the tag terminal detects a reflection signal, which is output from the tag in response to a signal output from the tag terminal, when the transfer unit transfers the patient to inside the magnet assembly and configured to transfer the diagnosis area of the patient to an optimum photographic area inside the magnet assembly based on the position information about the diagnosis area of the patient or the RF coil.

The tag includes a plurality of tags installed on the magnet assembly and the patient position determination system calculates distances of the RF coil or the diagnosis area of the patient with respect to each of the plurality of tags based on signals transmitted from the plurality of tags, and calculates a position of the diagnosis area of the patient or the RF coil by use of trigonometry.

A signal transmitted from the tag includes identification information about the tag, and the patient position determination system calculates the position of the diagnosis area, or the RF coil by identifying each of signals transmitted from the plurality of tags according to the identification information.

The tag terminal includes a signal transmitting unit configured to transmit signals and a signal receiving unit configured to receive signals that are transmitted from the tag.

In accordance with another aspect of the present invention, a control method of a magnetic resonance imaging device, which has a magnet assembly and a patient table provided with a transfer unit introducing a patient to inside the magnet assembly and provided with a fixing unit supporting the transfer unit, is as follows. If a signal, which is generated from a tag installed on a predetermined area of the magnet assembly in response to a signal transmitted from a tag terminal installed on a diagnosis area of the patient or a radio frequency (RF) coil attached to the diagnosis area, is received by the tag terminal, a distance between the tag and the tag terminal is calculated. A position of the diagnosis area of the patient or the RF coil is calculated based on the distance information. The diagnosis area of the patient is transferred to an optimum photographic area in the magnet assembly.

As described above, after a position of a diagnosis area of a patient is detected, the patient is transferred to the inside the magnet assembly of the magnetic resonance imaging device, thereby reducing the preparatory time.

### BRIEF DESCRIPTION OF THE DRAWINGS

These and/or other aspects of the invention will become apparent and more readily appreciated from the following description of the exemplary embodiments, taken in conjunction with the accompanying drawings of which:
FIG. 1 is a block diagram illustrating an exemplary embodiment of a magnetic resonance imaging device of the present invention.
FIG. 2 is a block diagram illustrating a gradient rotation and compensation processor of the exemplary embodiment of the magnetic resonance imaging device of the present invention shown in FIG. 1.
FIGS. 3A to 3D are views illustrating a magnet assembly of the exemplary embodiment of the magnetic resonance imaging device of the present invention.
FIG. 4 is a view illustrating an MRI photographing system configured to detect a position of a diagnosis area of a patient and to control the moved distance of a transfer unit in the exemplary embodiment of the magnetic resonance imaging device of the present invention.
FIGS. 5A to 5B are views illustrating the movement of a signal receiving unit receiving a signal with a narrow frequency band in the exemplary embodiment of the magnetic resonance imaging device of FIG. 4.
FIG. 5C is a graph showing the intensity of a signal according to the movement of the signal receiving unit in the exemplary embodiment of the magnetic resonance imaging device shown in FIGS. 5A-5B.
FIGS. 6A to 6C are views illustrating the movement of a signal receiving unit receiving a signal with a broad frequency band in the exemplary embodiment of the magnetic resonance imaging device of FIG. 4.
FIG. 6D is a graph showing the intensity of a signal according to the movement of the signal receiving unit in the exemplary embodiment of the magnetic resonance imaging device shown in FIGS. 6A-6C.
FIG. 7 is a view illustrating a signal transmitting unit installed on a predetermined area of the magnet assembly of the exemplary embodiment of the magnetic resonance imaging device of the present invention.
FIG. 8A is a view illustrating a signal receiving unit of the magnetic resonance imaging device that is connected to a patient position determination system through wireless connection.
FIG. 8B is a view illustrating a signal receiving unit of the magnetic resonance imaging device that is connected to a patient position determination system through wired connection.
FIG. 9 is a view illustrating another exemplary embodiment of a magnetic resonance imaging device of the present invention, in which a tag terminal is installed on a magnet assembly, and a tag is attached to a diagnosis area of a patient or an RF coil.
FIG. 10 is a view illustrating an example of a plurality of signal transmitting units installed in an alternative embodiment of a magnet assembly of a magnetic resonance imaging device of the present invention.
FIGS. 11A to 11D are views illustrating placement of a patient and controlling a moved distance of a transfer unit in a magnetic resonance imaging device.
FIG. 12 is a flowchart illustrating an exemplary embodiment of a control method of a magnetic resonance imaging device of the present invention.

### DETAILED DESCRIPTION OF THE INVENTION

Reference will now be made in detail to the preferred embodiments of the present invention, examples of which are illustrated in the accompanying drawings, wherein like reference numerals refer to like elements throughout. This invention may, however, be embodied in many different forms and should not be construed as limited to the exemplary embodiments set forth herein. In the following description, a detailed explanation of known related functions and constructions may be omitted to avoid unnecessarily obscuring the subject matter of the present invention. Also, terms described herein, which are defined considering the functions of the present invention, may be implemented differently depending on user and operator's intention and practice. Therefore, the terms should be understood on the basis of the disclosure throughout the specification. The principles and features of this invention may be employed in varied and numerous embodiments without departing from the scope of the invention.

Furthermore, although the drawings represent exemplary embodiments of the invention, the drawings are not necessarily to scale and certain features may be exaggerated or omitted in order to more clearly illustrate and explain the present invention.

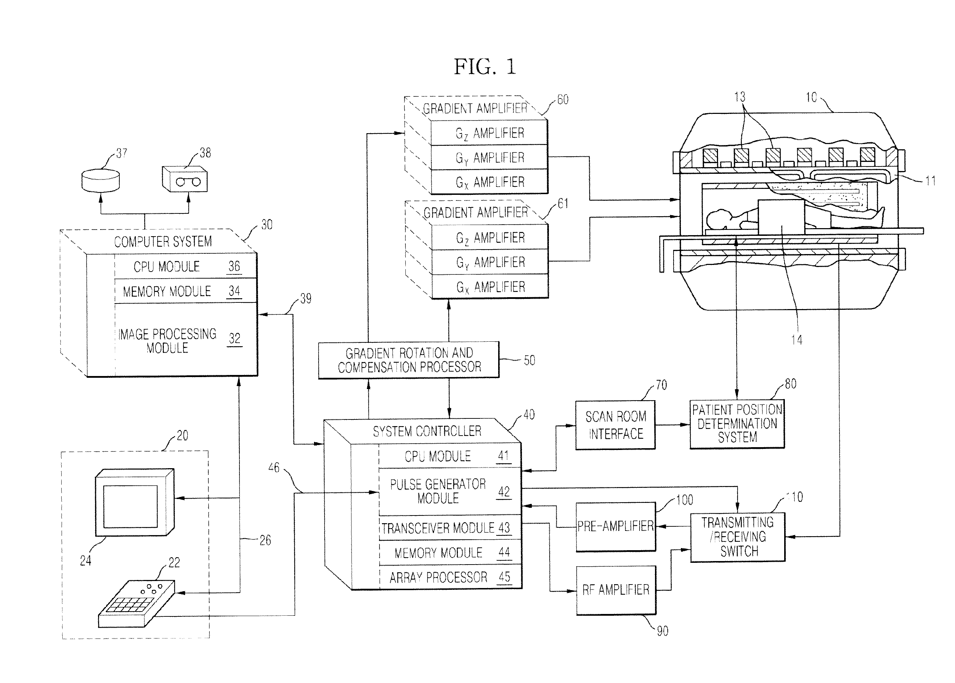

FIG. 1 is a block diagram illustrating an exemplary embodiment of a magnetic resonance imaging (MRI) device of the present invention.

As shown in FIG. 1, the magnetic resonance imaging device includes a magnet assembly 10, an operation console 20, a computer system 30, a system controller 40, a gradient rotation and compensation processor 50, gradient amplifiers 60 and 61, a scan room interface 70, a patient position determination system 80, an RF amplifier 90, a pre-amplifier 100 and a transmitting/receiving switch 110.

The magnet assembly 10 generates a static magnetic field in a longitudinal direction of a cylindrical bore, into which the patient is introduced, placed, or otherwise moved, and allows a gradient magnetic field to be added to the static magnetic field when an MRI photographing procedure is performed.

The operation console 20 may include a control panel 22 configured to allow an operator to operate the computer system 30, and a display 24 configured to represent a control state of the overall magnetic resonance imaging device. The operation console 20 is connected to the computer system 30 through a link 26, which may be a wired and/or wireless connection.

The computer system 30 enables an image to be displayed on the display 24 through operation of an operator, such as a user or technician. The computer system 30 may include a plurality of modules that communicate with each other through a backplane or other components. The modules include an image processing module 32, a memory module 34 and a CPU module 36. The computer system 30 is operatively connected to any known memory device for storing information. For example, the computer system 30 may be linked to a disk memory unit 37, which records image data and programs, and to a tape driver 38. The computer system 30 also communicates with a system controller 40 through a high speed serial link 39 which operatively connects the computer system 30 to the system controller 40. Alternatively, other known data communication systems and devices in addition to or instead of the high speed serial link 39 may be used to connect the computer system 30 with the system controller 40.

The system controller 40 includes a set of modules that are connected to one another through a backplane or connected using other known connection components. The set of modules includes a CPU module 41, a pulse generator module 42, a transceiver module 43, a memory module 44, and an array processor 45.

The pulse generator module 42 is connected to the operation console 20 through a serial link 46, and receives a command from an operator using the control panel 22, with the command representing a scan sequence of an MRI procedure to be initiated by the magnetic resonance imaging device shown in FIG. 1. The pulse generator module 42 operates respective components of the computer system 30 to execute a desired scan sequence. The pulse generator module 42 generates a gradient waveform that represents the timing, intensity, and direction of a magnetic field gradient that is produced during scanning. The gradient waveform generated from the pulse generator module 42 is applied to the gradient rotation and compensation processor 50.

The pulse generator module 42 may be connected to the scan room interface 70. The scan room interface 70 receives signals from sensors that are related to a state of the magnetic resonance imaging device.

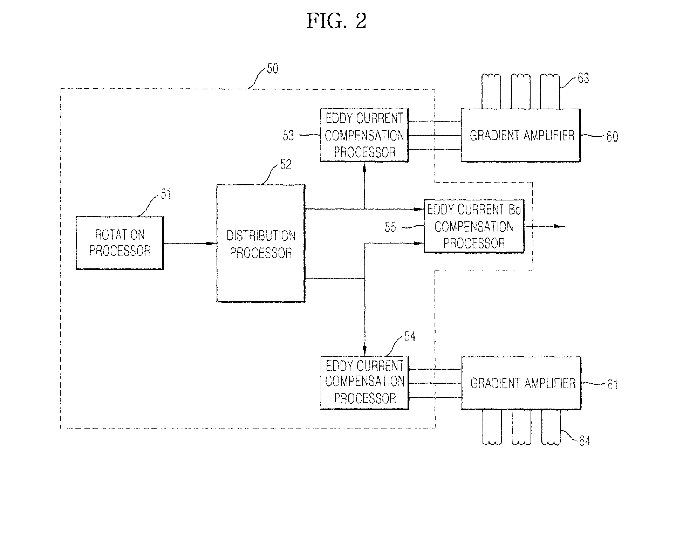

The pulse generator module 42 produces logical gradient waveforms that generate a slice-select, a phase encoding and a readout magnetic field gradient during a process of scanning. The logical gradient waveforms are transferred to the gradient rotation and compensation processor 50. Referring to FIG. 2, a rotation processor 51 of the gradient rotation and compensation processor 50 convert a logical gradient waveform to X, Y, and Z physical gradient waveforms. The logical gradient waveform is rotated in a space to generate gradient waveforms (Gₓ, G_{y}, and G_{z}) according to physical gradient axes X, Y, and Z. The physical gradient waveforms Gₓ, G_{y}, and G_{z} are transmitted to a distribution processor 52 that generates physical gradient waveforms corresponding to a first gradient coil set 63 and a second gradient coil set 64. The first gradient coil set 63 serves as a main coil set. The distribution processor 52 multiplies input physical gradient waveforms by a coefficient (C) that generates the optimum gradient field intensity for three main gradient coils included in a gradient coil set 11, shown in the magnet assembly 10 in FIG. 1. The distribution processor 52 multiples the input physical gradient waveforms by a coefficient (1-C), thereby distributing a remainder of the physical gradient waveforms to the second gradient coil set 64 that serves as a subsidiary gradient coil set. Eddy current compensation processors 53 and 54, connected to respective gradient amplifiers 60, 61, compensate for the physical gradient waveforms for the main gradient coil set and the subsidiary gradient coil set. An eddy current Bo compensation processor 55 receives a distributed gradient waveform from the distribution processor 52, and combines Bo compensation current into a single Bo compensation signal.

The gradient amplifiers 60 and 61 drive a gradient coil of the gradient coil set 11 to generate a magnetic field gradient used to perform a position encoding on a magnetic resonance imaging (MRI) signal, also known as a nuclear magnetic resonance (NMR) signal. The gradient coil set 11 includes the main gradient coil set driven by the gradient amplifier 60, and by the subsidiary gradient coil set driven by the gradient amplifier 61. The gradient coil set 11 serves as a part of the magnet assembly 10, and includes a polarized magnet 13, as shown in FIG. 1.

The transceiver module 43 of the system controller 40 generates pulses, which are amplified by an RF amplifier 90, and is connected to a radio frequency (RF) coil 14 positioned substantially adjacent to an intended diagnosis area D of the patient via the transmitting/receiving switch 110, as shown in FIG. 1. Accordingly, a final MRI signal, also known as a final NMR signal, which is emitted from excited nuclei of the body of the patient, is detected by the RF coil 14 and then transmitted to the pre-amplifier 100 via the transmitting/receiving switch 110.

The patient position determination system 80 determines the position of the patient who is introduced, placed, or otherwise moved to the inside the magnet assembly 10. The patient position determination system 80 performs control functions, such that the RF coil 14 is positioned substantially adjacent to the diagnosis area D of the patient, and so is disposed on or near the optimum area of the patient for MRI photographing and imaging.

The RF coil 14 receives the final MRI signal, also known as the final NMR signal, which is emitted from excited nuclei of the patient when the magnet assembly 10 generates the magnetic field. The received final MRI signal is converted to a digital signal and transmitted to the memory module 44. If the memory module 44 stores the entire data array obtained until the scanning is completed, the array processor 45 converts the stored data to an image data array through a Fourier transformation. The data processed through the above procedures is transmitted to the computer system 30 through the high speed serial link 39, and stored in the disk memory unit 37 or the tape drive 38.

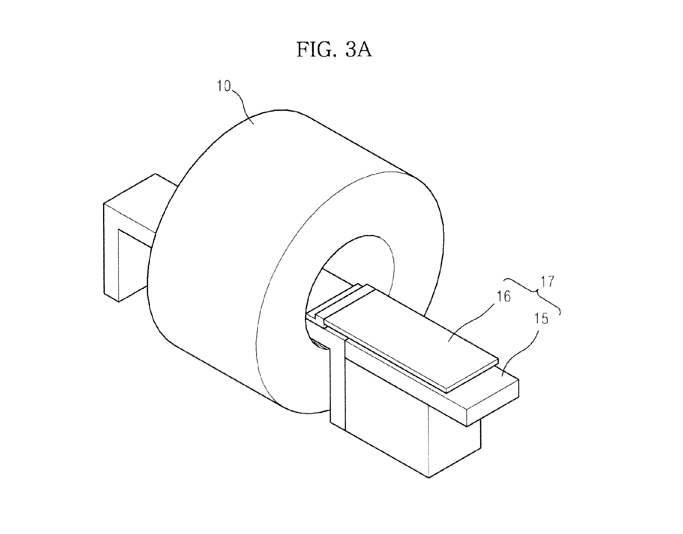

FIGS. 3A to 3D are views illustrating a magnet assembly 10 that is provided in the exemplary embodiment of the magnetic resonance imaging device of the present invention as shown in FIG. 1.

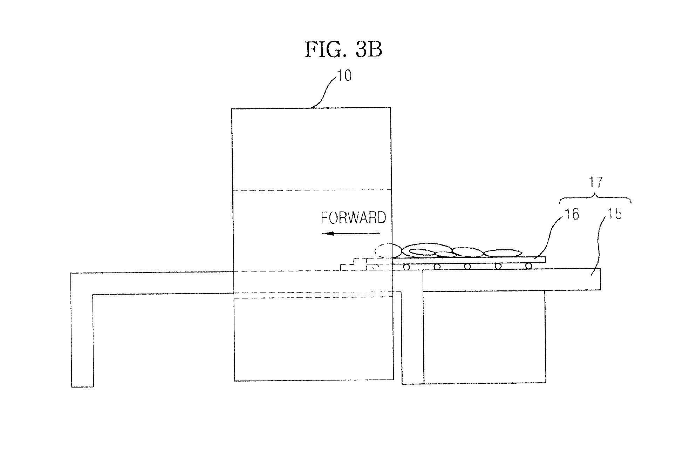

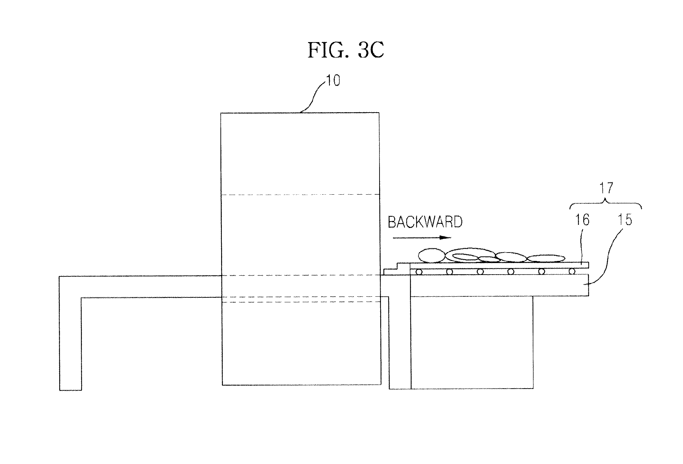

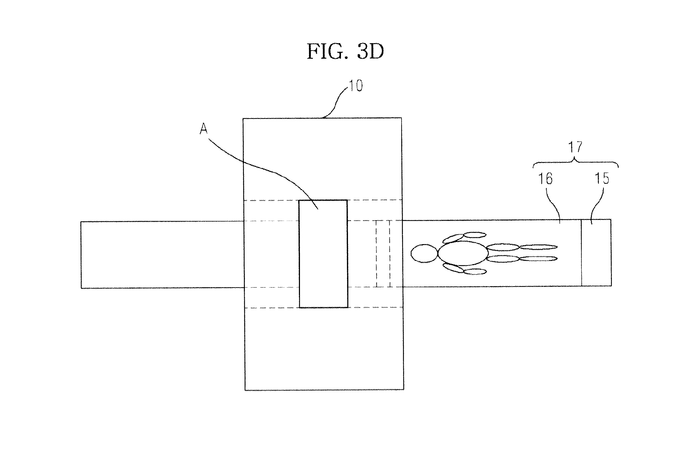

Referring to FIG. 3A in conjunction with FIG. 1, the magnetic resonance imaging device may include the magnet assembly 10 and a patient table 17. The patient table 17 includes a fixing unit 15 and a transfer unit 16. The transfer unit 16 is insertable into the inside the magnet assembly 10. Referring to FIGS. 3B and 3C, the transfer unit 16 moves backward and forward parallel to the longitudinal axis of the magnet assembly 10 while having the patient lying thereon. In one example embodiment, the transfer unit 16 is positioned atop rollers, shown in FIGS. 3B-3C, to slide longitudinally relative to the fixing unit 15. However, it is understood that other known devices may be used to provide the transfer unit 16 with a sliding engagement with the fixing unit 15. The transfer unit 16 moves backward to unload the patient and moves forward while having another patient loaded thereon to perform MRI photographing. At this time, the RF coil 14 is installed on the diagnosis area D of the patient, and the diagnosis area D having the RF coil 14 installed thereon needs to be positioned at an optimum area for MRI photographing in the magnet assembly 10. Referring to FIG. 3D, the diagnosis area D of the patient having the RF coil 14 installed thereon needs to be positioned at or substantially near a predetermined area (A) that is optimal for MRI photographing inside the magnet assembly 10. In positioning the diagnosis area D of the patient at or substantially near the predetermined area (A), MRI photographing systems in the prior art require an additional operation of marking a position while moving the transfer unit 16 of the patient table 17. Such an operation increases the MRI photographing time of the magnetic resonance imaging device, and degrades or lowers the efficiency of the magnetic resonance imaging device. However, according to the exemplary embodiment of the magnetic resonance imaging device of the present invention, the moved distance of the transfer unit 16 is automatically adjusted by detecting a position of the diagnosis area D of the patient. Hereinafter, an example of automatically adjusting the moved distance of the transfer unit 16 is described with reference to accompanying drawings. As defined herein, the optimum area A for MRI photographing is a location in the magnet assembly 10 in which the diagnosis area D is exposed to the strongest flux of magnetic fields, including the gradient magnetic fields and the static magnetic fields, from the magnet assembly. In the exemplary embodiments of the present invention, the optimum area A is located at or substantially near the center of the magnetic assembly 10.

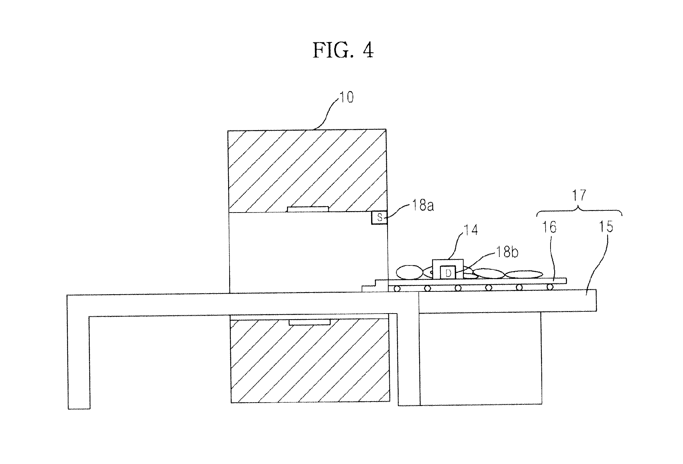

FIG. 4 is a view illustrating an MRI photographing system configured to detect a position of the diagnosis area D of the patient, and to control the moved distance of a transfer unit in the exemplary embodiment of the magnetic resonance imaging device of the present invention.

The magnetic resonance imaging device includes a signal transmitting unit 18a, labeled S in FIG. 4, which is provided at an entrance of the magnet assembly 10; for example, inside the magnet assembly 10. The magnetic resonance imaging device also includes a signal receiving unit 18b, which is installed on the diagnosis area D of the patient. The signal transmitting unit 18a transmits signals, such as optical signals, ultrasonic signals, and RF signals. The signal receiving unit 18b detects signals transmitted from the signal transmitting unit 18a, and transmits detection information to the patient position determination system 80. The signal transmitted from the signal transmitting unit 18a may be a wide range of signals including a signal with a narrow frequency band or a signal with a broad frequency band. The available bandwidth of a signal may be determined in advance during a process of designing the magnetic resonance imaging device.

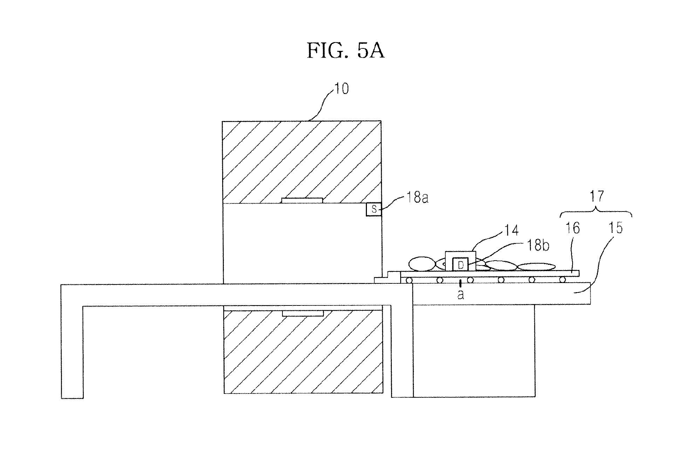

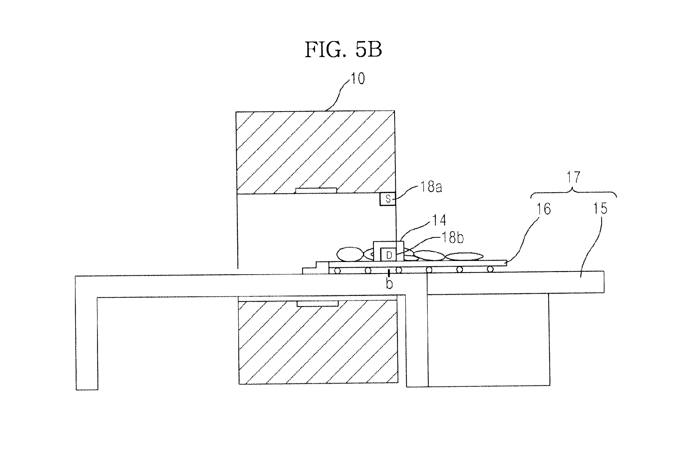

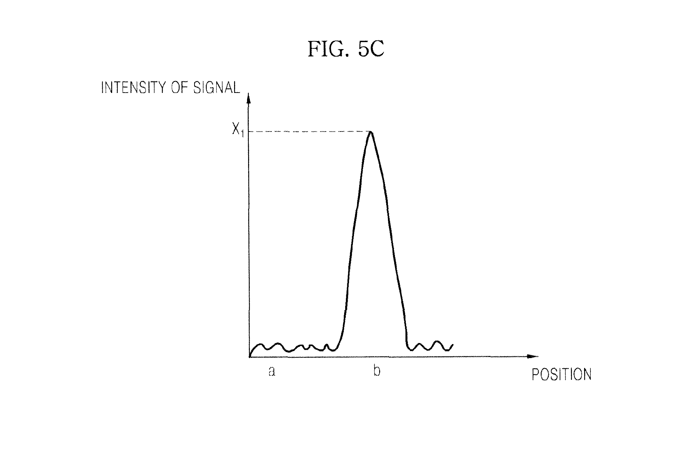

In the case that the signal transmitting unit 18a is determined to transmit a signal with a narrow frequency band, the patient position determination system 80 estimates that the RF coil 14 or the diagnosis area D of the patient is vertically below the position of the signal transmitting unit 18a at a point of time in which the signal sending unit 18b receives a signal. Referring to FIGS. 5A to 5C, when the RF coil 14 installed on the patient or the diagnosis area D of the patient is positioned substantially near or just above an area 'a', shown in FIG. 5A, the intensity of a received signal is almost or substantially equal to zero, as shown in FIG. 5C. When the RF coil 14 installed on the patient or the diagnosis area D of the patient is positioned substantially near or just above an area 'b', shown in FIG. 5B, the intensity of a received signal is equal to the value X₁, as shown in FIG. 5C. Accordingly, the patient position determination system 80 estimates that the RF coil 14 or the diagnosis area D of the patient is disposed at the entrance of the magnet assembly 10 at a point of time in which the intensity of a signal is measured to be X₁, and controls the moved distance of the transfer unit 16. The patient position determination system 80 has information about an initial position of the RF coil 14 or the diagnosis area D of the patient, and controls the operational speed of the transfer unit 16, thereby transferring the RF coil 14, and the associated diagnostic area D of the patient, to the optimum MRI photographic area in the magnet assembly 10.

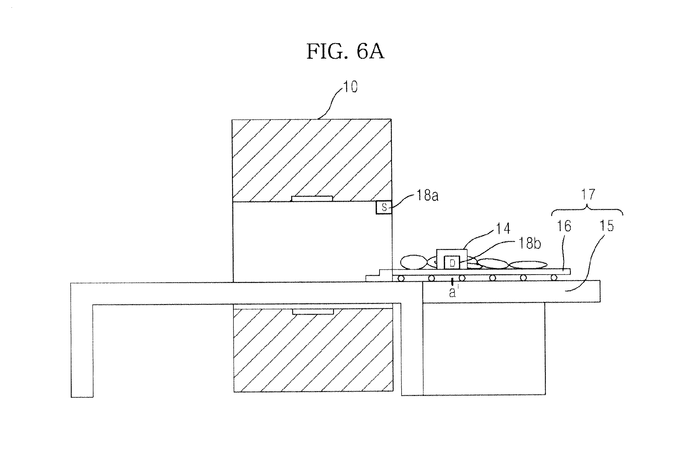

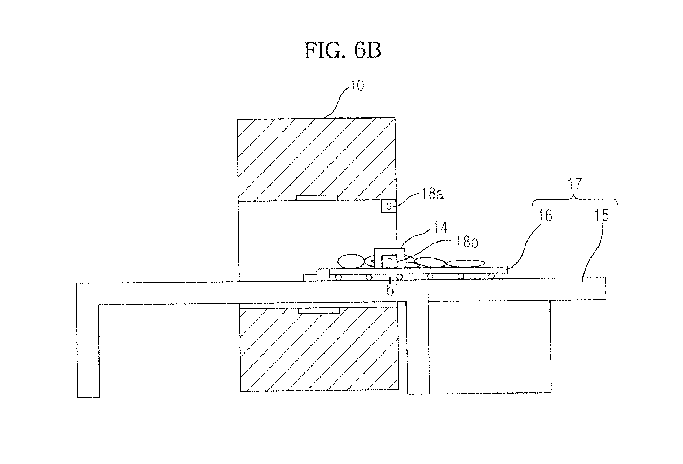

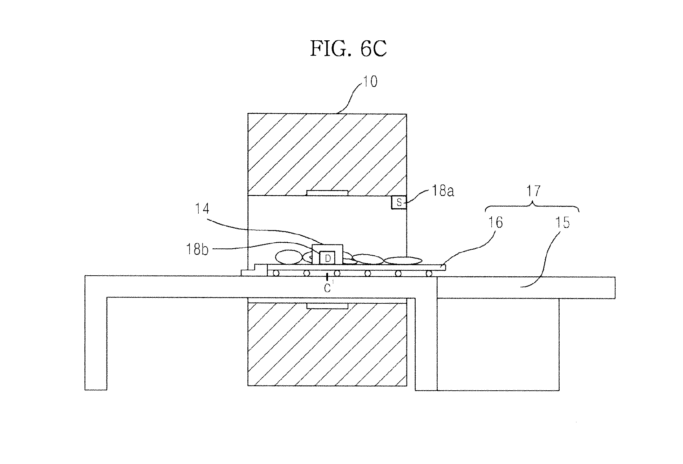

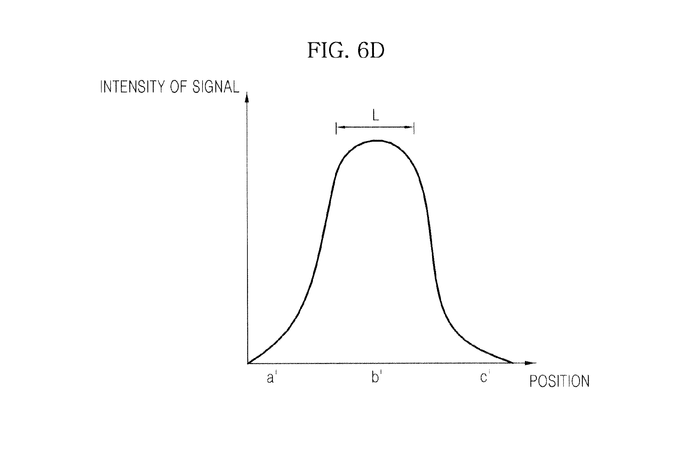

In the case that the signal transmitting unit 18a is determined to transmit a signal with a broad frequency band, the patient position determination system 80 estimates the position of the RF coil 14 or the diagnosis area D of the patient based on the intensity of a signal received by the signal receiving unit 18b. Referring to FIGS. 6A to 6D, the intensity of signal gradually increases when the RF coil 14 or the diagnosis area D of the patient moves from a position a' to a position b', and gradually decreases when the RF coil 14 or the diagnosis area D of the patient moves from the position b' to a position c', as shown in the graph in FIG. 6D. In this case, the patient position determination system 80 estimates the RF coil 14 or the diagnosis area D of the patient is disposed vertically below the signal transmitting unit 18a in an area or range of longitudinal positions 'L' within the magnet assembly 10 in which the intensity of a signal changes from increasing to decreasing, as shown in FIG. 6D.

In this manner, the patient position determination system 80 estimates the position of the RF coil 14 or the diagnosis area D of the patient, and controls the operational speed and operating time of the transfer unit 16 such that the RF coil 14, and the associated diagnostic area D of the patient, is disposed within the optimum

MRI photographic area inside the magnet assembly 10.

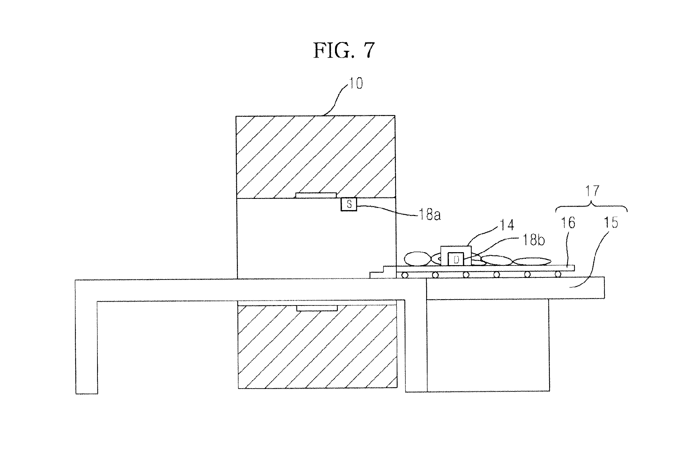

Meanwhile, referring to FIG. 7, the signal transmitting unit 18a may be disposed at a predetermined area or location other than the entrance of the magnet assembly 10. The patient position determination system 80 has information about the predetermined location of the signal transmitting unit 18a within the magnet assembly 10 where the signal transmitting unit 18a is installed. Accordingly, at a point of time in which a signal is received or the greatest intensity of a signal is measured by the signal receiving unit 18b, the patient position determination system 80 estimates that the signal receiving unit 18b is disposed at or substantially near a region or location corresponding to the signal transmitting unit 18a. However, in the case that the signal transmitting unit 18a is determined to transmit a signal with a narrow frequency band and also only if the signal transmitting unit 18a is installed between the optimum MRI photographing area inside the magnet assembly 10 and inside the entrance of the magnet assembly 10, the moved distance of the transfer unit 16 is controlled based on the detected position.

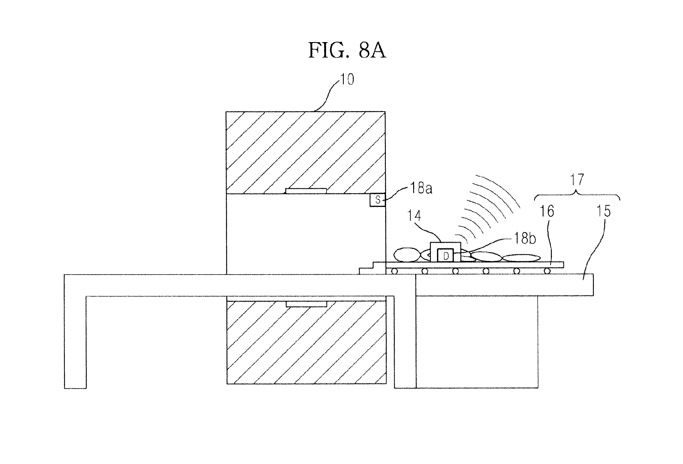

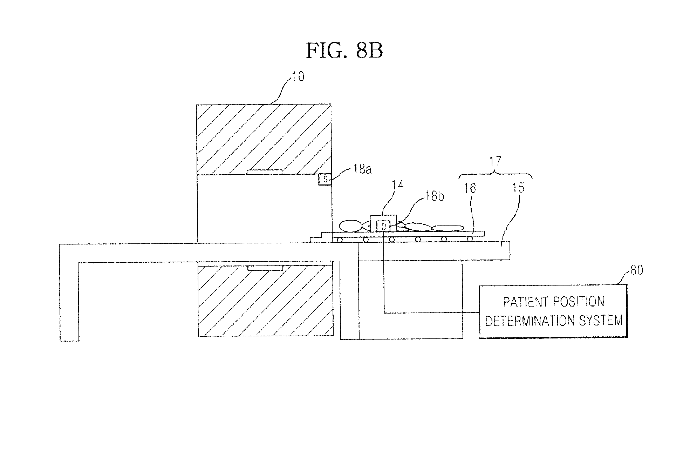

Referring to FIGS. 8A and 8B, the signal receiving unit 18b may be connected to the patient position determination system 80 through a wireless connection, as shown in FIG. 8A, or a wired connection, as shown in FIG. 8B.

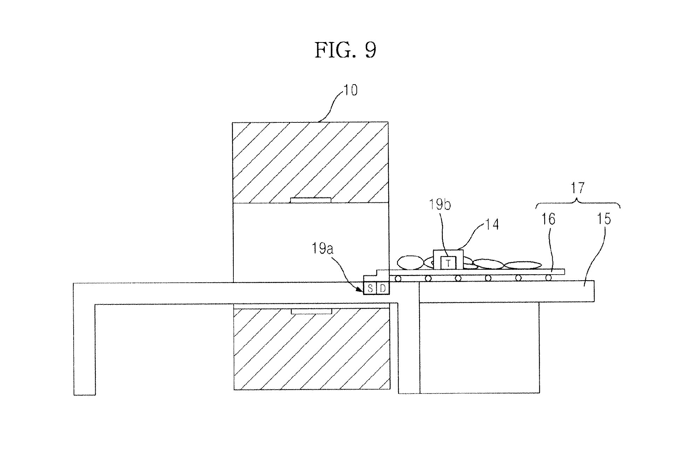

FIG. 9 is a view illustrating another exemplary embodiment of a magnetic resonance imaging device of the present invention, in which the position of the diagnosis area of the patient is detected, the moved distance of a transferring unit is controlled by use of a tag terminal 19b installed on the magnet assembly 10, and a tag 19a, having a label T in FIG. 9, is attached to the diagnosis area.

The tag 19a is installed on the RF coil 14 or the diagnosis area of the patient, and the tag terminal 19b is installed on the magnet assembly 10. The tag terminal 19b transmits a predetermined signal and receives a response signal, which is reflected from the tag 19a. If the tag 19a is positioned substantially above or near an area adjacent to the tag terminal 19b, the tag 19a transmits a predetermined response signal corresponding to the predetermined signal transmitted from the tag terminal 19b. The tag terminal 19b receives the response signal transmitted from the tag 19a, and transmits corresponding received information to the patient position determination system 80. Upon reception of the response signal transmitted from the tag 19a, the patient position determination system 80 estimates that the RF coil 14 or the diagnosis area of the patient has reached a position at or near the entrance of the magnet assembly 10, and controls the position of the transfer unit 16. The tag 19a may be implemented using an RF tag. The tag terminal 19b may be implemented using a radio frequency identification (RFID) terminal. The RF tag receives the predetermined signal, which is transmitted from the RFID terminal, generates a response signal including tag information, and transmits the generated response signal to the RFID terminal. The RFID terminal receives the response signal, which is reflected by the RF tag, and transmits the received information to the patient position determination system 80. In a preferred embodiment, the RF tag and RFID terminal transmit RF signals on frequencies substantially different from the RF signals used by the magnetic resonance imaging device for MRI photographing; for example, the MRI photographing is performed in different RF bands than the RF tag and RFID terminal, in order to avoid interference in the MRI photographing by the RF tag and RFID terminal.

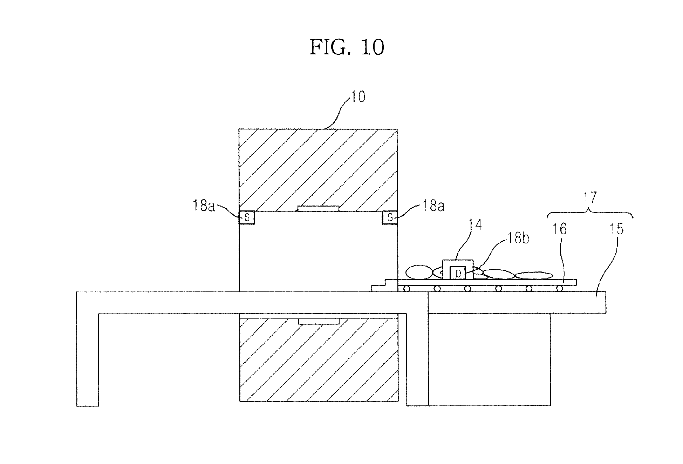

FIG. 10 is a view illustrating an example of a plurality of signal transmitting units installed in an alternative embodiment of the magnet assembly of the magnetic resonance imaging device of the present invention.

The magnet assembly 10 may be provided with a plurality of signal transmitting units 18a, each labeled S in FIG. 10. The signal transmitting units 18a may adjust the output time of transmitted signals such that the transmitted signals are distinguished from one another; that is, the signal transmitting units 18a may alternately transmit signals. The signal receiving unit 18b installed on the RF coil 14 or the diagnosis area D of the patient may determine the origin of the signals based on the reception time of the signals.

In addition, the plurality of signal transmitting units 18a may simultaneously output signals. In this case, each of the signals transmitted from the signal transmitting units 18a may include identification information that indicates its origin. For example, each transmitted signal may include identification information about a respective signal transmitting unit 18a, and so processing of such transmitted signals, received by the signal receiving unit 18b, by, for example, the system controller 40, with reference to predetermined stored locations of each of the signal transmitting units 18a, may be used to determine the location of the diagnosis area D relative to signal transmitting units 18a and the position of the diagnosis area D relative to the magnet assembly 10.

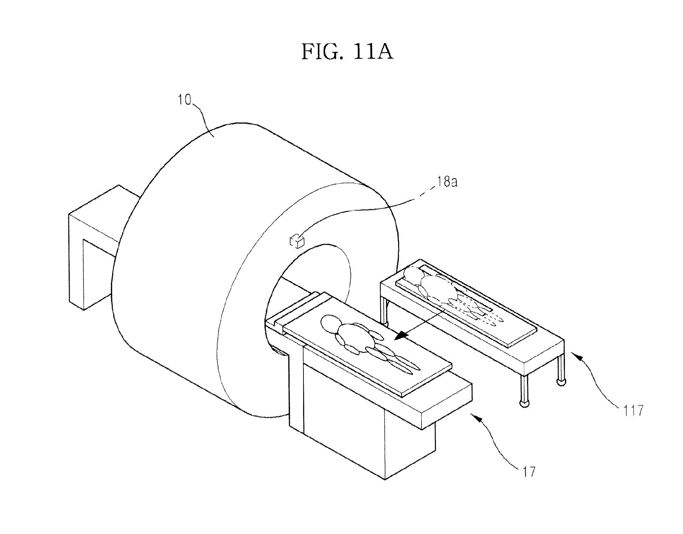

FIGS. 11A to 11D are views illustrating an example of placement of a patient on the transfer unit 16, and controlling a moved distance of the transfer unit 16 in the magnetic resonance imaging device.

Referring to FIG. 11A, the patient is moved from a movable patient table 117 to the patient table 17 of the magnetic resonance imaging device.

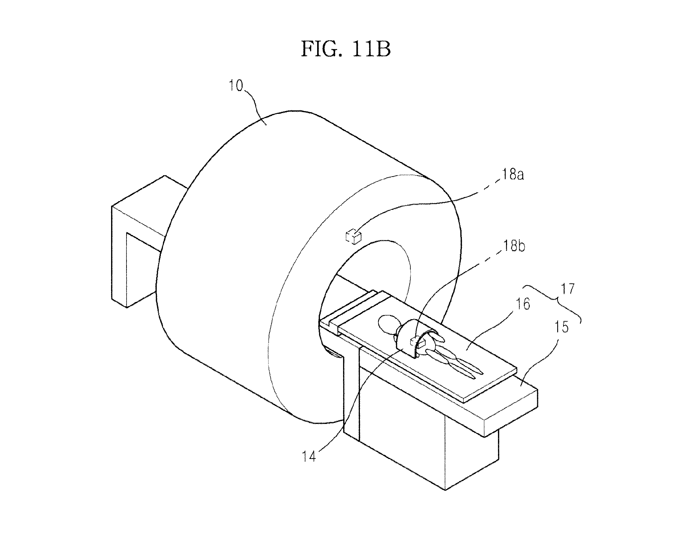

Referring to FIG. 11 B, the RF coil 14 is installed on or substantially adjacent to the diagnosis area D of the patient. The RF coil 14 or the diagnosis area D of the patient has the signal receiving unit 18b attached thereto. The signal receiving unit 18b is connected to the patient position determination system 80 through a wireless connection or a wired connection.

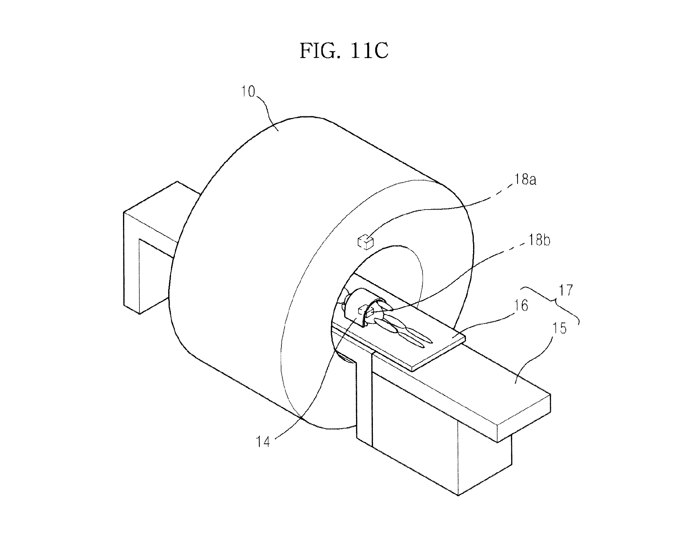

Referring to FIG. 11C, the transfer unit 16 of the patient table 17 moves under the control of the patient position determination system 80. The signal receiving unit 18b receives a signal output from the signal transmitting unit 18a installed at a predetermined area or location or in an entrance area on the inside the magnet assembly 10, and transmits the received signal to the patient position determination system 80. The patient position determination system 80 estimates the position of the RF coil 14 or the diagnosis area D of the patient based on information about the signal received by the signal receiving unit 18b, and adjusts the moved distance of the transfer unit 16 based on the estimated position.

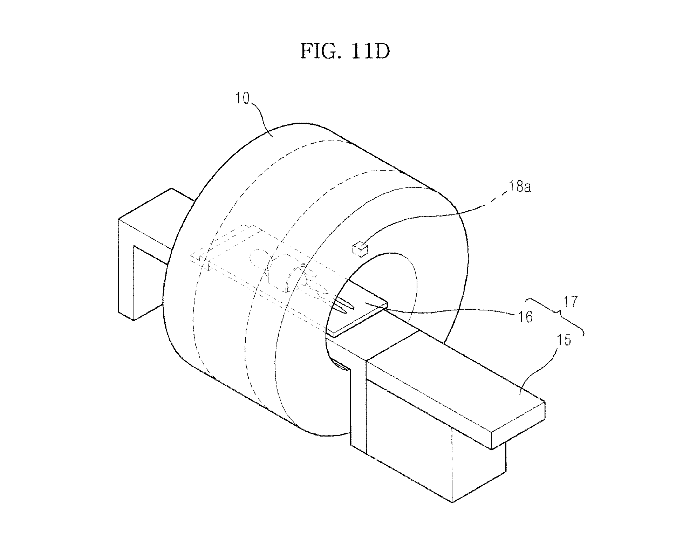

Referring to FIG. 11 D, after the RF coil 14 is positioned at or substantially adjacent to an optimum MRI photographing area in the magnet assembly 10, the MRI photographing is performed on the diagnosis area D of the patient.

Meanwhile, the operation of alternative embodiments of the magnetic resonance imaging device of the present invention, in which a tag is installed on a magnet assembly and a tag terminal is attached to the diagnosis area D of the patient or the RF coil 14, is substantially identical to that described with reference to FIGS. 11A to 11D.

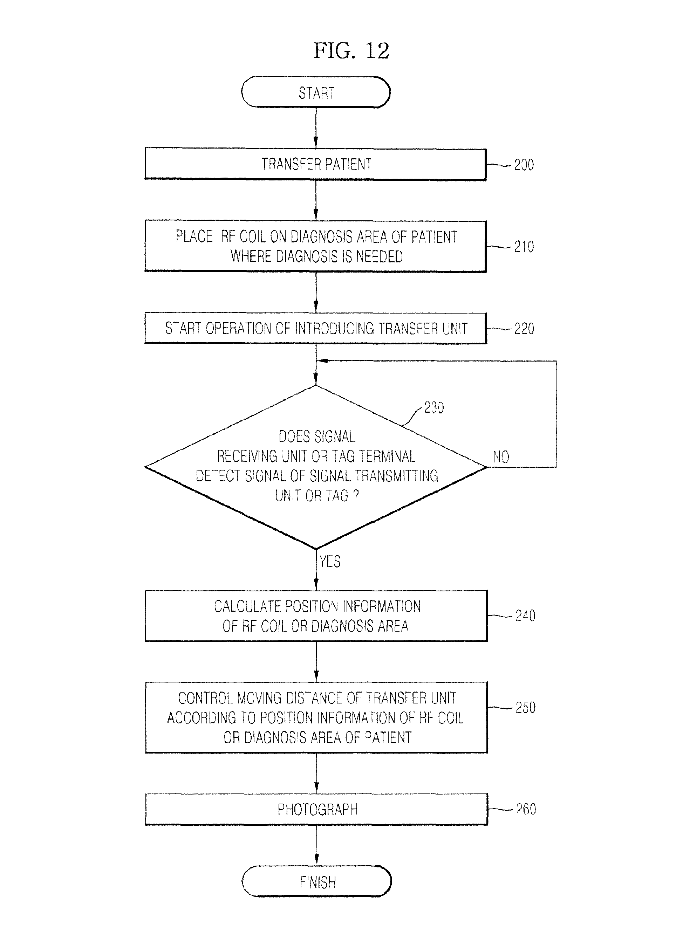

FIG. 12 is a flowchart illustrating an exemplary embodiment of a control method of a magnetic resonance imaging device of the present invention, shown in various exemplary embodiments in FIGS. 1-11D.

An operator of a magnetic resonance imaging device transfers the patient to the patient table 17 of the magnetic resonance imaging device in step 200; for example, the patient is transferred from a separate movable patient table 117, as shown in FIG. 11 B, or is pushed in a wheelchair to the patient table 17, or otherwise, the patient is escorted to the patient table 17 by an aide, or lies on the patient table without assistance.

The operator or other staff of the magnetic resonance imaging device installs the RF coil 14 on the diagnosis area D of the patient in step 210. The signal receiving unit 18b, for example, as shown in the various drawings herein, or the tag 19a, for example, as shown in various drawings herein, may be installed on the RF coil 14 or on the diagnosis area D of the patient. If the signal receiving unit 18b is installed, a single signal transmitting unit 18a, or alternatively a plurality of such signal receiving units 18b, as described herein, are installed inside the magnet assembly 10. If the tag 19a is installed or used, the tag terminal 19b is installed inside the magnet assembly 10, as described herein.

The patient position determination system 80 starts an operation of introducing, placing, or otherwise moving the transfer unit 16, with the patient thereon, into the magnet assembly 10 in step 220 after the RF coil 14 is installed on the diagnosis area D. The transfer unit 16 transfers the patient to the inside of the magnet assembly 10 to perform MRI photographing of the patient.

The patient position determination system 80 checks, in step 230, whether a signal is received by the signal receiving unit 18b or the tag terminal 19b that is installed on the RF coil 14 or the diagnosis area D of the patient. The signal received by the signal receiving unit 18b or the tag terminal 19b is a signal output from the signal transmitting unit 18a or the tag 19a, respectively. If the signal transmitting unit 18a is designed to output a signal with a narrow frequency band, the patient position determination system 80 of the present invention determines that the RF coil 14 or the diagnosis area D of the patient is positioned vertically below the signal transmitting unit 18a at a point of time in which the signal receiving unit 18b receives a signal, as in FIG. 5C. If the signal transmitting unit 18a is designed to output a signal having a broad frequency band, the patient position determination system 80 of the present invention determines that the RF coil 14 or the diagnosis area D of the patient is positioned vertically below the signal transmitting unit 18a at a point of time in which the intensity of a signal changes from increasing to decreasing, as in FIG. 6D. In addition, if a plurality of signal transmitting units 18a or a plurality of tags 19a are installed inside the magnet assembly 10, the distances of the RF coil 14 or the diagnosis area D of the patient, which has the signal receiving unit 18b or the tag terminal 19b installed thereon, with respect to each of the signal transmitting units 18a or each of the tags 19a, respectively, are calculated, and the position of the RF coil 14 or the diagnosis area D of the patient is calculated by use of trigonometry in step 240, for example, using predetermined equations or tables of values for determining the position of the RF coil 14 or the diagnosis area D from the specific time of receipt of such signals from the signal transmitting units 18a and/or tags 19a, and from the locations of the signal receiving units 18b and/or tag terminals 19b.

The patient position determination system 80 controls the moved distance of the transfer unit 16 in step 250, such that the diagnosis area D of the patient reaches the optimum MRI photographing area, according to the position information of the RF coil 14 or the diagnosis area D of the patient determined in step 240. Thereafter, with the patient and/or the diagnosis area D of the patient being properly positioned at an optimum location within the magnet assembly 10 for accurate MRI photographing, an MRI photographing procedure is performed on the patient in step 260.

The above description has been made for embodiments in which the signal transmitting unit 18a or the tag terminal 19b are installed inside the magnet assembly 10. However, the installation position of the signal transmitting unit 18a or the tag terminal 19b is not limited thereto. The signal transmitting unit 18a or the tag terminal 19b may be installed at an entrance of the magnet assembly 10 or outside the magnet assembly 10 to transmit signals for implementing the present invention to properly position the patient and/or the diagnosis area D of the patient within the magnet assembly 10 for accurate MRI photographing. In addition, the placement of signal transmitting units 18a or tag terminals 19b is not limited to being above the patient. It is understood that such signal transmitting units 18a or tag terminals 19b may be placed in any orientation about the patient within the magnet assembly 10, and the system controller 40 determines distances to the diagnosis area D accordingly. Therefore, the present invention is not limited to detecting when signal transmitting units 18a or tag terminals 19b are positioned vertically above the diagnosis area D.

The above-described apparatus and methods according to the present invention can be implemented in hardware, firmware or as software or computer code that can be stored in a recording medium such as a CD ROM, a RAM, a floppy disk, a hard disk, or a magneto-optical disk or computer code downloaded over a network originally stored on a remote recording medium or a non-transitory machine readable medium and to be stored on a local recording medium, so that the methods described herein can be rendered in such software that is stored on the recording medium using a general purpose computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein.

Although a few embodiments of the present invention have been shown and described, it would be appreciated by those skilled in the art that changes may be made in these embodiments without departing from the principles and spirit of the invention, the scope of which is defined in the claims and their equivalents.

## Claims

1. A magnetic resonance imaging (MRI) device which has a magnet assembly and a table provided with a transfer unit for moving an object to inside the magnet assembly, and with a fixing unit supporting the transfer unit, the magnetic resonance imaging device comprising:
a signal transmitting unit installed on the magnet assembly;
a signal receiving unit installed on a diagnosis area of the object or a radio frequency (RF) coil attached to the diagnosis area of the object; and
a position determination system configured to calculate position information of the diagnosis area of the object or the RF coil if the signal receiving unit detects a signal output from the signal transmitting unit when the transfer unit moves the object to inside of the magnet assembly and to move the diagnosis area of the object to a predetermined MRI imaging area inside the magnet assembly based on the position information of the diagnosis area of the object or the RF coil.

2. The magnetic resonance imaging device of claim 1, wherein the signal transmitting unit comprises a plurality of signal transmitting units that are installed on the magnet assembly, and wherein, in response to the signal receiving unit receiving signals from the plurality of signal transmitting units, the position determination system calculates distances of the RF coil or the diagnosis area of the object, which has the signal receiving unit installed thereon, with respect to each of the plurality of signal transmitting units, and calculates a position of the RF coil or the diagnosis area of the object based on the calculated distance information.

3. The magnetic resonance imaging device of claim 2, wherein, in response to the position of the RF coil or the diagnosis area of the object being calculated, the position determination system calculates a distance between the position of the RF coil or the diagnosis area of the object and the predetermined MRI imaging area in the magnetic assembly and moves the diagnosis area of the object to the predetermined MRI imaging area in the magnet assembly.

4. The magnetic resonance imaging device of claim 1, wherein the signal transmitting unit outputs a signal with a broad frequency band or a signal with a narrow frequency band,
and if the signal transmitting unit outputs a signal with a narrow frequency band, the position determination system determines that the signal transmitting unit is positioned substantially vertically above the signal receiving unit when the signal receiving unit receives the signal.

5. The magnetic resonance imaging device of claim 4, wherein if the signal transmitting unit outputs a signal with a broad frequency band, the position determination system determines that the signal transmitting unit is positioned substantially vertically above the signal receiving unit when an intensity of the signal received by the signal receiving unit changes from increasing to decreasing.

6. A control method of a magnetic resonance imaging device (MRI) which has a magnet assembly and a table provided with a transfer unit for moving an object to inside the magnet assembly, and with a fixing unit supporting the transfer unit, the control method comprising:
checking whether a signal transmitted from a signal transmitting unit installed on a predetermined area of the magnet assembly is received by a signal receiving unit, which is installed on a diagnosis area of the object or a radio frequency (RF) coil attached to a diagnosis area of the object, when the object is moved into the magnet assembly;
responsive to the signal transmitted from the signal transmitting unit being received by the signal receiving unit, calculating a position of the diagnosis area of the object or the RF coil based on information about the received signal; and
moving the diagnosis area of the object to a predetermined MRI imaging area in the magnet assembly.

7. The control method of claim 6, wherein the signal transmitting unit comprises a plurality of signal transmitting units installed on the magnet assembly, wherein distances of the signal receiving unit with respect to each of the plurality of signal transmitting units are calculated, and a position of the RF coil or the diagnosis area of the object is calculated based on the calculated distance information.

8. The control method of claim 6, wherein the signal transmitting unit outputs a signal with a narrow frequency band, and a position determination system determines that the signal transmitting unit is positioned substantially vertically above the diagnosis area of the object or the RF coil when the signal receiving unit receives the signal.

9. The control method of claim 6, wherein the signal transmitting unit outputs a signal having a broad frequency band, and a position determination system determines that the signal transmitting unit is positioned substantially vertically above the diagnosis area of the object or the RF coil when an intensity of the signal received by the signal receiving unit changes from increasing to decreasing.

10. A magnetic resonance imaging (MRI) device which has a magnet assembly and a table provided with a transfer unit for moving an object to inside the magnet assembly, and with a fixing unit supporting the transfer unit, the magnetic resonance imaging device comprising:
a tag installed on a predetermined area of the magnet assembly;
a tag terminal installed on a diagnosis area of the object or a radio frequency (RF) coil attached to a diagnosis area of the object; and
a position determination system configured to calculate position information about the diagnosis area of the object or the RF coil if the tag terminal detects a reflection signal, which is output from the tag in response to a signal output from the tag terminal, when the transfer unit moves the object to inside the magnet assembly, and configured to move the diagnosis area of the object to a predetermined MRI imaging area inside the magnet assembly based on the position information about the diagnosis area of the object or the RF coil.

11. The magnetic resonance imaging device of claim 10, wherein the tag comprises a plurality of tags installed on the magnet assembly, and the position determination system calculates distances of the RF coil or the diagnosis area of the object with respect to each of the plurality of tags based on signals transmitted from the plurality of tags, and calculates a position of the diagnosis area of the object or the RF coil by use of a predetermined equation.

12. The magnetic resonance imaging device of claim 11, wherein the signals transmitted from the plurality of tags comprises identification information about each respective tag, and the position determination system calculates the position of the diagnosis area, or the RF coil by identifying each of the signals transmitted from the plurality of tags according to the identification information.

13. The magnetic resonance imaging device of claim 10, wherein the tag terminal comprises a signal transmitting unit configured to transmit signals and a signal receiving unit configured to receive signals that are transmitted from the tag.

14. A control method of a magnetic resonance imaging (MRI) device which has a magnet assembly and a table provided with a transfer unit for moving an object to inside the magnet assembly, and with a fixing unit supporting the transfer unit, the control method comprising:
responsive to a signal, which is generated from a tag installed on a predetermined area of the magnet assembly in response to a signal transmitted from a tag terminal installed on a diagnosis area of the object or a radio frequency (RF) coil attached to the diagnosis area, being received by the tag terminal, calculating a distance between the tag and the tag terminal;
calculating a position of the diagnosis area of the object or the RF coil based on the distance information; and
moving the diagnosis area of the object to a predetermined MRI imaging area in the magnet assembly.

15. A magnetic resonance imaging (MRI) device which has a magnet assembly and a table provided with a transfer unit for moving an object to inside the magnet assembly, and with a fixing unit supporting the transfer unit, the magnetic resonance imaging device comprising:
a tag terminal installed on a predetermined area of the magnet assembly;
a tag installed on a diagnosis area of the object or a radio frequency (RF) coil attached to a diagnosis area of the object; and
a position determination system configured to calculate position information about the diagnosis area of the object or the RF coil if the tag terminal detects a reflection signal, which is output from the tag in response to a signal output from the tag terminal, when the transfer unit moves the object to inside the magnet assembly, and configured to move the diagnosis area of the object to a predetermined MRI imaging area inside the magnet assembly based on the position information about the diagnosis area of the object or the RF coil.
